# EUROPEAN PATENT APPLICATION

(11) **EP 3 858 389 A1**
(43) Date of publication of application: **04.08.2021**
(21) Application number: 21153794.9
(22) Date of filing: 27.01.2021
(51) Int. Cl.: A61L 2/00, A61L 2/02, A61L 2/04, A61L 2/10, A61L 2/20, A61L 2/24

(54) **HAND DISINFECTING APPARATUS AND METHOD FOR OPERATION**

(30) Priority: 27.01.2020 NO 20200104
(71) Applicant: Smart Cleaner AS, 6770 Nordfjordeid (NO)
(72) Inventor: Erling, Bjørlo, 6770 Nordfjordeid (NO)
(74) Representative: Basck Ltd

(57) **Abstract**

There is provided a hand disinfecting apparatus including a housing, wherein the housing includes an access arrangement to receive one or more user's hands into hand-receiving chamber of hand disinfecting apparatus, characterized in that when hand disinfecting apparatus, when deployed for operation, has its internal elements disposed, for example, from top to bottom: an ozone source at a top region of hand disinfecting apparatus that adds ozone to air flow through ozone source; an electrical heater that receives air flow from the ozone source; a hand-receiving chamber that receives user's hands and passes air flow including heated ozone-enriched air over user's hands; a filter arrangement that receives air flow of heated ozone-enriched air after it has passed over the user's hands, wherein filter arrangement collects pathogens from passed air flow; and a fan arrangement that provides for air flow from top region to bottom region of hand disinfecting apparatus, wherein the fan arrangement includes ozone absorbing arrangement.

## Description

### TECHNICAL FIELD

The present disclosure relates generally to cleansing apparatus; more specifically, the present disclosure relates to hand disinfecting apparatus that, when in operation, dry and disinfect (for example sterilize) hands of a given user. Moreover, the present disclosure also relates to methods for operating aforesaid hand disinfecting apparatus.

### BACKGROUND

In everyday life, human hands come into contact with many surfaces from which bacteria and other pathogens are susceptible to being collected onto the human hands. Although conventional hand washing, for example involving use of antiseptic soaps and clean water, kills a significant portion of such bacteria and other pathogens, such hand washing is not effective to avoid completely a risk of spreading infection. With very resilient bacteria such as "superbugs" (for example, MRSA: methicillin-resistant Staphylococcus aureus) as well as highly contagious pathogens such as the Ebola virus (Zaire ebolavirus, causing a severe and often fatal haemorrhagic fever in humans and other mammals), such hand washing is insufficient when medical staff move between highly-sterile zones (for example, isolation areas) and a general non-sterile environment. For example, when surgeons and associated operating theatre support staff are to enter an operating theatre, it is essential to try to avoid importing bacteria and other pathogens into the operating theatre, especially in view of increasing antibiotic resistance amongst many strains of common bacteria.

In the recent past, advancements have been made in respect of cleansing techniques; such advancements relate to, for example, an addition of hot air being blown along with cleansing gas such as ozone. However, such conventional techniques also do not attain potential efficacy due to inefficient controlling techniques.

Hand sterilizing apparatus are known. For example, in a granted patent GB2502268 (Applicant: Heat Outdoors Ltd.), there is described a combination hand dryer/air sterilizer apparatus comprising:
(i) a housing unit;
(ii) a hand dryer unit comprising:
   (a) a duct having an input opening and an output opening;
   (b) a fan unit disposed in fluid communication with said duct for generating an air flow from said output opening;
   (c) a heater disposed in fluid communication with said duct for heating said airflow;
   (d) a control circuit for energising said fan unit and said heater in response to an actuation signal; and
(iii) an air sterilization unit;
wherein both the air sterilizing unit and the hand dryer unit are housed within the same housing unit. Optionally, the air sterilization unit comprises a sterilization unit which operates using one or more of the processes in the group of processes comprising: Ozone generation; Photoplasma generation; Negative ion generation; Deep-UV-light; and Photo Catalytic Oxidation. Optionally the air sterilization unit comprising a catalyst and a plurality of UV lamps.

In a granted European patent EP2693927B1 (Applicant: Excel Dryer Inc.), there is described a sanitizing hand dryer, comprising:
(i) a dryer housing having an air inlet and an exit nozzle;
(ii) an air filter assembly having a coarse filter and a high-efficiency particulate air filter that receives air from the air inlet and provides filtered air, where the coarse filter and the high-efficiency particulate air filter are serially arranged with the coarse filter upstream of the high-efficiency particulate air filter;
(iii) a blower that draws filtered air and accelerates that filtered air to provide high speed filtered air; and
wherein the sanitizing hand dryer further comprises an ion generator that includes a wire grid through which the high speed filtered air passes to provide sanitized air to the exit nozzle.

The aforementioned a combination hand dryer/air sterilizer apparatus and the aforementioned sanitizing hand dryer are not able to sterilize users' hands sufficiently for many situations where complete sterilization of dangerous bacteria and pathogens is required.

Therefore, in light of the foregoing discussion, there exists a need to overcome the aforementioned drawbacks associated known hand sterilizing apparatus.

### SUMMARY

The present disclosure seeks to provide an improved hand disinfecting apparatus (for example, an improved hand sterilizing apparatus) to disinfect the user's hands in a highly efficient manners by removal of harmful pathogens (such as Ebola, Tuberculosis, and the like).

The present disclosure also seeks to provide a method for operating the hand disinfecting apparatus to disinfect the user's hands.

In a first aspect, the present disclosure provides a hand disinfecting apparatus including a housing, wherein the housing includes a central region having an access arrangement to receive one or more user's hands into a hand-receiving chamber of the hand disinfecting apparatus, characterized in that
when the hand disinfecting apparatus, when deployed for operation, has its internal elements disposed, from a first region within the housing to a second region within the housing:
(a) an ozone source at the first region within the housing of the hand disinfecting apparatus that adds ozone to an air flow through the ozone source;
(b) an electrical heater that receives the air flow from the ozone source, wherein the air flow includes ozone-enriched air and heats the received air flow including ozone-enriched air to generate an air flow including heated ozone-enriched air;
(c) the hand-receiving chamber that receives the one or more user's hands and passes the air flow including heated ozone-enriched air over the one or more user's hands;
(d) a filter arrangement that receives the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) a fan arrangement that provides for the air flow that is drawn from the first region to the region second of the hand disinfecting apparatus, wherein the fan arrangement includes an ozone absorbing arrangement.

The invention is of advantage in that the present disclosure provides a hand disinfecting apparatus (for example, a hand sterilizing apparatus) that, for example, is able to identify the pathogen type and destroy the same by the sequential processing of the internal elements as defined hereinabove. Moreover, the hand disinfecting apparatus is able to reduce or avoid generating disposable materials such as soiled paper towels and soap deposits that could potentially harbour pathogens; the hand disinfecting apparatus is thus able to reduce generating dangerous waste that potentially includes pathogens.

Beneficially, optionally, in the hand disinfecting apparatus, the first region is a top region of the housing and the second region is a bottom region of the housing, when the hand disinfecting apparatus is in operation. An advantage of such an arrangement is that water drips from the hand receiving chamber fall under an effect of gravity and of the air flow through the hand receiving chamber onto the filter arrangement.

Alternatively, optionally, in the hand disinfecting apparatus, the first region is a bottom region of the housing and the second region is a top region of the housing, when the hand disinfecting apparatus is in operation.

Yet alternatively, optionally, in the hand disinfecting apparatus, the first region and the second region are regions of the housing that are spatially lateral to the hand receiving chamber, when the hand disinfecting apparatus is in operation.

Yet alternatively, optionally, in the hand disinfection apparatus, one of the first and second regions is a region of the housing that is spatially lateral to the hand receiving chamber, and another of the first and second regions is spatially vertical to the hand receiving chamber, when the hand disinfecting apparatus is in operation.

Optionally, in the hand disinfecting apparatus, the ozone source is arranged spatially vertically relative to the electrical heater, when the hand disinfecting apparatus is in operation.

Optionally, in the hand disinfecting apparatus, the ozone source is arranged spatially laterally relative to the electrical heater, when the hand disinfecting apparatus is in operation.

Optionally, in the hand disinfecting apparatus, the fan arrangement is arranged spatially vertically relative to the filter arrangement, when the hand disinfecting apparatus is in operation.

Optionally, in the hand disinfecting apparatus, the fan arrangement is arranged spatially laterally relative to the fan arrangement, when the hand disinfecting apparatus is in operation.

In another aspect, the present disclosure provides a method of operating a hand disinfecting apparatus (for example, a hand sterilizing apparatus) including a housing, wherein the housing includes in a central region within the housing an access arrangement to receive one or more user's hands into a hand-receiving chamber of the hand disinfecting apparatus, characterized in that
the method includes steps of:
(a) using an ozone source disposed at a first region within the housing of the hand disinfecting apparatus to add ozone to an air flow through the ozone source;
(b) using an electrical heater that receives the air flow from the ozone source, wherein the air flow includes ozone-enriched air and heats the received air flow including ozone-enriched air, to generate an air flow including heated ozone-enriched air;
(c) using the hand-receiving chamber to receives the one or more user's hands and to pass the air flow including heated ozone-enriched air over the one or more user's hands;
(d) using a filter arrangement to receive the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) using a fan arrangement to provide for the air flow to be drawn from the first region to a second region within the housing of the hand disinfecting apparatus, wherein the fan arrangement includes an ozone absorbing arrangement.

Beneficially, optionally, in the method, the first region is a top region of the housing and the second region is a bottom region of the housing, when the hand disinfecting apparatus is in operation. An advantage of such an arrangement is that water drips from the hand receiving chamber fall under an effect of gravity and of the air flow through the hand receiving chamber onto the filter arrangement.

Alternatively, optionally, in the method, the first region is a bottom region of the housing and the second region is a top region of the housing, when the hand disinfecting apparatus is in operation.

Yet alternatively, optionally, in the method, the first region and the second region are regions of the housing that are spatially lateral to the hand receiving chamber, when the hand disinfecting apparatus is in operation.

Yet alternatively, optionally, in the hand disinfecting apparatus, one of the first and second regions is a region of the housing that is spatially lateral to the hand receiving chamber, and another of the first and second regions is spatially vertical to the hand receiving chamber, when the hand disinfecting apparatus is in operation.

Optionally, in the method, the ozone source is arranged spatially vertically relative to the electrical heater, when the hand disinfecting apparatus is in operation.

Optionally, in the method, the ozone source is arranged spatially laterally relative to the electrical heater, when the hand disinfecting apparatus is in operation.

Optionally, in the method, the fan arrangement is arranged spatially vertically relative to the filter arrangement, when the hand disinfecting apparatus is in operation.

Optionally, in the hand disinfecting apparatus, the fan arrangement is arranged spatially laterally relative to the fan arrangement, when the hand disinfecting apparatus is in operation.

Beneficially, when in use, a given user firstly washes his or her hands in clean water for circa 30 seconds, and then inserts his or her hands into the hand disinfecting apparatus for 30 seconds, wherein the given user's hands are disinfected (for example, sterilized) and dry.

In yet another aspect, an embodiment of the present disclosure provides a computer program product comprising a non-transitory computer-readable storage medium having computer-readable instructions stored thereon, the computer-readable instructions being executable by a computerized device comprising processing hardware to execute a method for (namely, a method of) operating a hand disinfecting apparatus.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:

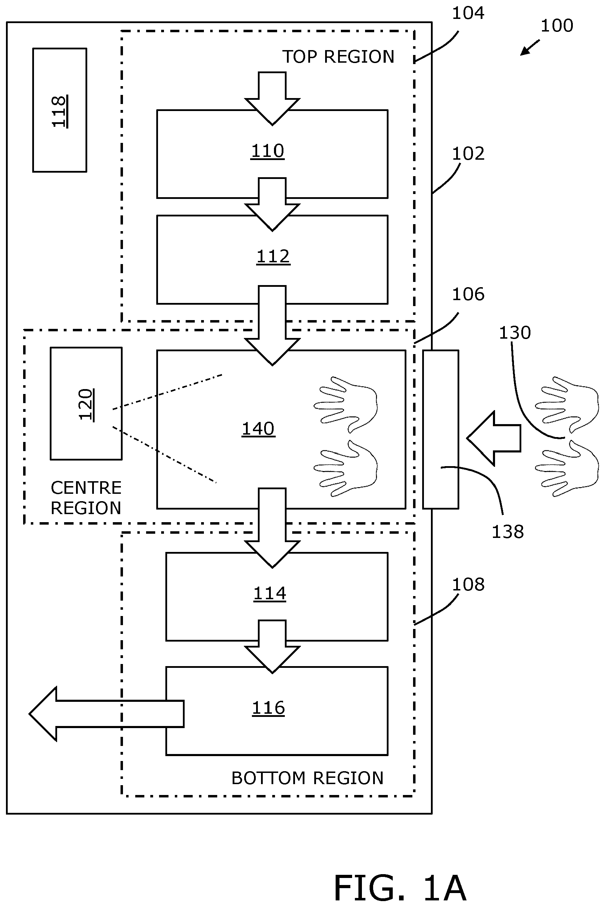

- FIG. 1A: is a schematic illustration of a hand disinfecting apparatus (for example, a hand sterilizing apparatus), in accordance with an embodiment of the present disclosure, wherein an ozone source and an electrical heater are disposed at a top region of a housing of the hand disinfecting apparatus, a hand receiving chamber is disposed at a central region of the housing, and a filter arrangement and an extractor fan are disposed at a bottom region of the housing, when the hand disinfecting apparatus is in operation;

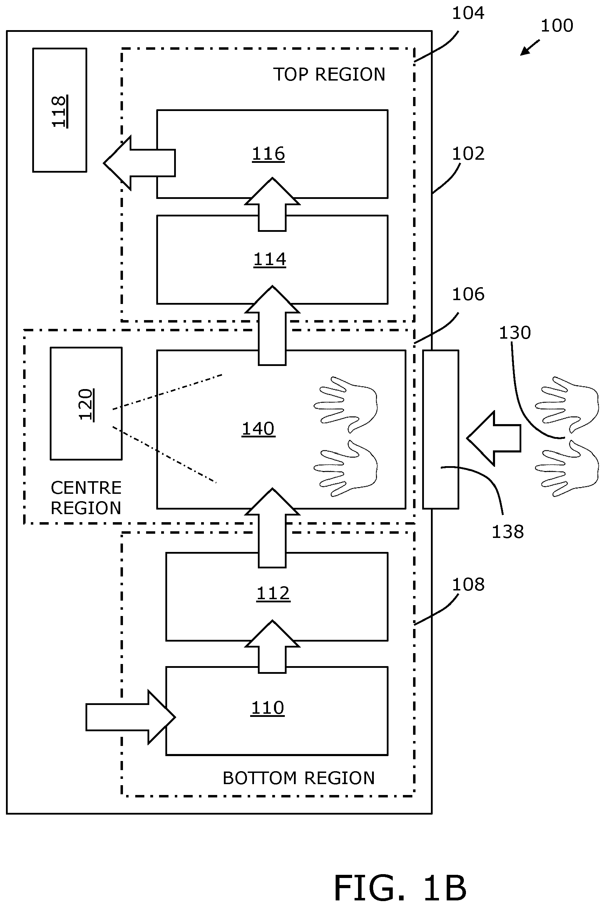

- FIG. 1B: is a schematic illustration of a hand disinfecting apparatus (for example, a hand sterilizing apparatus), in accordance with an embodiment of the present disclosure, wherein an ozone source and an electrical heater are disposed at a bottom region of a housing of the hand disinfecting apparatus, a hand receiving chamber is disposed at a central region of the housing, and a filter arrangement and an extractor fan are disposed at a top region of the housing, when the hand disinfecting apparatus is in operation;

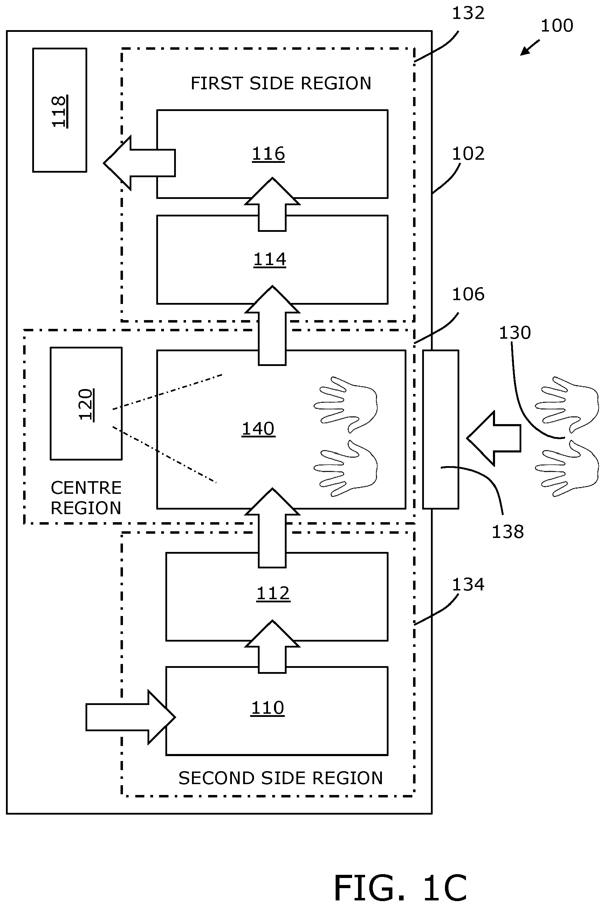

- FIG. 1C: is a schematic illustration of a hand disinfecting apparatus (for example, a hand sterilizing apparatus), in accordance with an embodiment of the present disclosure, wherein an ozone source and an electrical heater are disposed at a first lateral region of a housing of the hand disinfecting apparatus, a hand receiving chamber is disposed at a central region of the housing, and a filter arrangement and an extractor fan are disposed at a second lateral region of the housing, when the hand disinfecting apparatus is in operation;

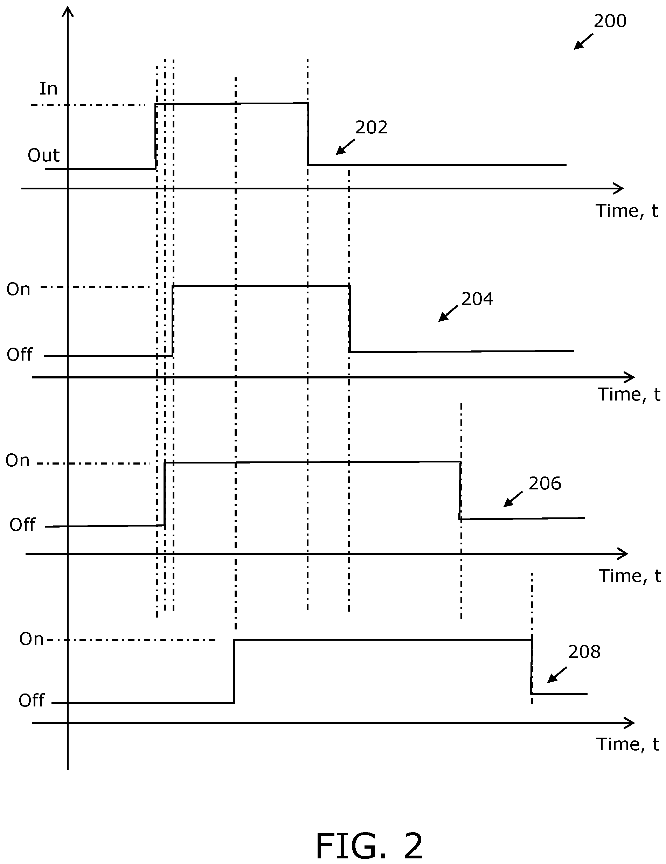

- FIG. 2: is a process-time graph of a sequence of activating component parts of the hand disinfecting apparatus of FIG. 1 when in use to dry and disinfect a user's hands of the hand disinfecting apparatus, in accordance with an embodiment of the present disclosure; and

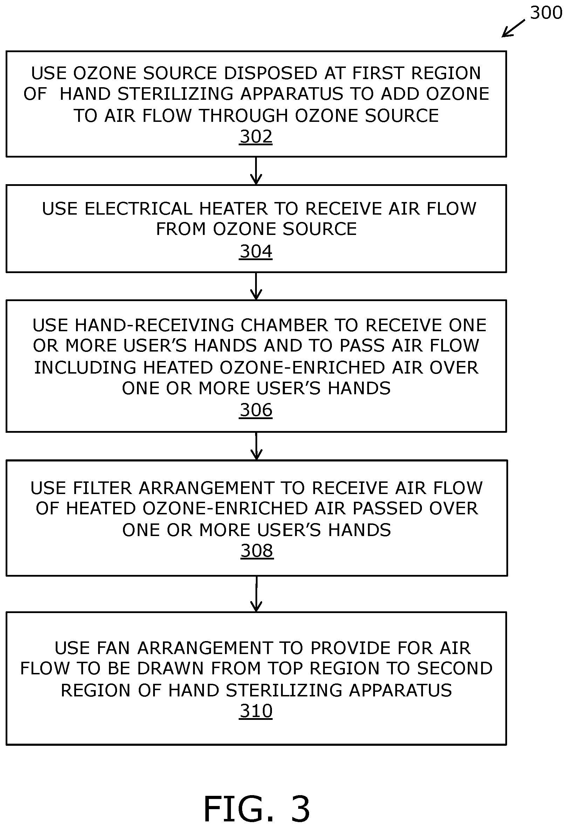

- FIG. 3: is an illustration of steps of a method for operating the hand disinfecting apparatus, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In a first aspect, the present disclosure provides a hand disinfecting apparatus including a housing, wherein the housing includes a central region having an access arrangement to receive one or more user's hands into a hand-receiving chamber of the hand disinfecting apparatus, characterized in that
when the hand disinfecting apparatus, when deployed for operation, has its internal elements disposed, from a first region within the housing to a second region within the housing:
(a) an ozone source at the first region within the housing of the hand disinfecting apparatus that adds ozone to an air flow through the ozone source;
(b) an electrical heater that receives the air flow from the ozone source, wherein the air flow includes ozone-enriched air and heats the received air flow including ozone-enriched air to generate an air flow including heated ozone-enriched air;
(c) the hand-receiving chamber that receives the one or more user's hands and passes the air flow including heated ozone-enriched air over the one or more user's hands;
(d) a filter arrangement that receives the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) a fan arrangement that provides for the air flow that is drawn from the first region to the region second of the hand disinfecting apparatus, wherein the fan arrangement includes an ozone absorbing arrangement.

Beneficially, optionally, in the hand disinfecting apparatus, the first region is a top region of the housing and the second region is a bottom region of the housing, when the hand disinfecting apparatus is in operation. An advantage of such an arrangement is that water drips from the hand receiving chamber fall under an effect of gravity and of the air flow through the hand receiving chamber onto the filter arrangement.

Alternatively, optionally, in the hand disinfecting apparatus, the first region is a bottom region of the housing and the second region is a top region of the housing, when the hand disinfecting apparatus is in operation. Such an arrangement benefits that the air flow when heated by the electrical heater, by way of convection, more easily moves upwards within the hand disinfecting apparatus.

Yet alternatively, optionally, in the hand disinfecting apparatus, the first region and the second region are regions of the housing that are spatially lateral to the hand receiving chamber, when the hand disinfecting apparatus is in operation. Such an implementation makes it possible for the hand disinfecting apparatus to be deployed as a table-top mounted unit.

Yet alternatively, optionally, in the hand disinfecting apparatus, one of the first and second regions is a region of the housing that is spatially lateral to the hand receiving chamber, and another of the first and second regions is spatially vertical to the hand receiving chamber, when the hand disinfecting apparatus is in operation.

Optionally, in the hand disinfecting apparatus, the ozone source is arranged spatially vertically relative to the electrical heater, when the hand disinfecting apparatus is in operation.

Optionally, in the hand disinfecting apparatus, the ozone source is arranged spatially laterally relative to the electrical heater, when the hand disinfecting apparatus is in operation. Such an arrangement of the hand disinfecting apparatus is potentially more spatially compact.

Optionally, in the hand disinfecting apparatus, the fan arrangement is arranged spatially vertically relative to the filter arrangement, when the hand disinfecting apparatus is in operation.

Optionally, in the hand disinfecting apparatus, the fan arrangement is arranged spatially laterally relative to the fan arrangement, when the hand disinfecting apparatus is in operation. Such an arrangement of the hand disinfecting apparatus is potentially more spatially compact.

In another aspect, the present disclosure provides a method of operating a hand disinfecting apparatus (for example, a hand sterilizing apparatus) including a housing, wherein the housing includes in a central region within the housing an access arrangement to receive one or more user's hands into a hand-receiving chamber of the hand disinfecting apparatus, characterized in that
the method includes steps of:
(a) using an ozone source disposed at a first region within the housing of the hand disinfecting apparatus to add ozone to an air flow through the ozone source;
(b) using an electrical heater that receives the air flow from the ozone source, wherein the air flow includes ozone-enriched air and heats the received air flow including ozone-enriched air, to generate an air flow including heated ozone-enriched air;
(c) using the hand-receiving chamber to receives the one or more user's hands and to pass the air flow including heated ozone-enriched air over the one or more user's hands;
(d) using a filter arrangement to receive the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) using a fan arrangement to provide for the air flow to be drawn from the first region to a second region within the housing of the hand disinfecting apparatus, wherein the fan arrangement includes an ozone absorbing arrangement.

Beneficially, optionally, in the method, the first region is a top region of the housing and the second region is a bottom region of the housing, when the hand disinfecting apparatus is in operation. An advantage of such an arrangement is that water drips from the hand receiving chamber fall under an effect of gravity and of the air flow through the hand receiving chamber onto the filter arrangement.

Alternatively, optionally, in the method, the first region is a bottom region of the housing and the second region is a top region of the housing, when the hand disinfecting apparatus is in operation.

Yet alternatively, optionally, in the method, the first region and the second region are regions of the housing that are spatially lateral to the hand receiving chamber, when the hand disinfecting apparatus is in operation.

Yet alternatively, optionally, in the method, one of the first and second regions is a region of the housing that is spatially lateral to the hand receiving chamber, and another of the first and second regions is spatially vertical to the hand receiving chamber, when the hand disinfecting apparatus is in operation.

Optionally, in the method, the ozone source is arranged spatially vertically relative to the electrical heater, when the hand disinfecting apparatus is in operation.

Optionally, in the method, the ozone source is arranged spatially laterally relative to the electrical heater, when the hand disinfecting apparatus is in operation.

Optionally, in the method, the fan arrangement is arranged spatially vertically relative to the filter arrangement, when the hand disinfecting apparatus is in operation.

Optionally, in the hand disinfecting apparatus, the fan arrangement is arranged spatially laterally relative to the fan arrangement, when the hand disinfecting apparatus is in operation.

Beneficially, the filter arrangement collects all of the one or more pathogens such that pathogen-free air is output from the filter arrangement. For example, the filter arrangement includes a plurality of filter mesh membranes arranged in a stack through which the air flow received from the hand receiving chamber is drawn by the fan arrangement.

In a third aspect, the present disclosure provides a computer program product comprising a non-transitory computer-readable storage medium having computer-readable instructions stored thereon, the computer-readable instructions being executable by a computerized device comprising processing hardware to execute a method for operating a hand disinfecting apparatus.

The present disclosure provides a hand disinfecting apparatus (for example, a hand sterilizing apparatus) for disinfecting users' hands. Furthermore, the hand disinfecting apparatus includes an ozone source to destroy the bacteria and pathogens on the users' hands with increased efficiency. Additionally, the hand disinfecting apparatus also includes the electrical heater to kill the bacteria and pathogens. The hand disinfecting apparatus also includes a filter arrangement to collect the residual bacteria and pathogens rinsed from the users' hands. The hand disinfecting apparatus further includes the control unit to control remotely the processing of the hand disinfecting apparatus.

An aerosol aqueous mist including polypeptides bound to fluorophores are employed in the hand disinfecting apparatus, wherein the aerosol mist selectively binds to portions of the filter arrangement where various types pathogens have collected; the polypeptides are selective in an affinity to bind to cell outer walls of specific corresponding types of pathogens. There is employed a UVC sterilizing source that illuminates the filter arrangement and causes the fluorophores to be stimulated and emit light at a preferred fluorophore light frequency. An colour imaging sensor (e.g. a camera) generates a series of images as the polypeptides bind to their preferred region of the filter arrangement, wherein analysis in computer hardware of the series of images provides an indication of a pathogen load being experienced by the filter arrangement. Optionally, different polypeptides have their unique corresponding fluorophores with associated emission wavelengths. Alternatively, optionally, the aerosol spray apparatus sprays a sequence of different polypeptides in the aerosol as a function of time, and the sequence of images records corresponding emissions from the filter arrangement when stimulated with UVC radiation from the UVC sterilizing source. Optionally, there is provided a wired or wireless, or both, data link from the hand disinfecting apparatus to a central database (not shown). When a hospital has many of the hand disinfecting apparatus, each hand disinfecting apparatus can be remotely monitored and a spread of pathogens within the hospital effectively monitored.

Beneficially, the hand disinfecting apparatus further includes the imaging sensor to collect the sequential images to provide an identification of pathogen type and pathogen load.

Throughout the present disclosure, the term "*hand disinfecting apparatus*" refers to cleansing device that substantially eliminates (for example, completely eliminates as in sterilization) bacteria and other pathogens, that otherwise lead to infections and various health problems such as diarrhoea, nausea, infections, diseases and so forth. The hand disinfecting apparatus used herein includes a housing having the access arrangement to receive one or both of users' hands into the hand-receiving chamber of the hand disinfecting apparatus. It will be appreciated that the housing may refer to a casing that houses various components of the hand disinfecting apparatus. Furthermore, the hand disinfecting apparatus, when deployed for operation, has its internal elements disposed, from top to bottom as aforementioned. Furthermore, the internal elements of the hand disinfecting apparatus are disposed in a manner that the sequential arrangement and operation thereof results in an efficient cleansing of the users' hands. Moreover, the hand disinfecting apparatus disinfects the skin and eliminates bacteria and pathogens through using a chain of processes such as spraying fluid, blowing air, and so forth. Particularly, for example, the air is circulated from top to the bottom of the hand disinfecting apparatus via various processes to allow a sanitization of the users' hands.

In an embodiment, the hand disinfecting apparatus includes a tall metal casing with four planar stainless-steel sides, a flat top and a flat base. Approximately half-way up one side is an opening where a user can insert his or her hands (after washing the hands) for drying and disinfection (for example, sterilization) purposes; the opening can be provided with a flap door and motion-actuated door (e.g. using ultrasonic or optical sensors) to avoid a need for a user to make physical contact with the door (thereby, reducing a risk of pathogens being left on the door by contact). The hand disinfecting apparatus is intended for use in hospitals and also field sites where contamination by pathogens is to be limited (for example, an Ebola treatment centre). The tall metal casing has a height of circa 0.9 metres, and lateral width of circa 40 cm, and a depth of circa 30 cm.

The hand disinfecting apparatus includes, for example, an ozone source at a top region of the hand disinfecting apparatus that adds ozone to an air flow through the ozone source. The ozone as used herein refers to the ozone gas that acts as a disinfectant that kills the microbes and pathogens. Beneficially, the ozone is dispensed in an efficient quantity that does not react with the skin and creates no harm to the skin. The ozone source adds ozone to the flow of air being circulated from top to bottom, that mix up with the air and flows in the direction of the air. For example, the ozone is infused in the flowing air to enhance the cleansing potential of the air and thereby increasing the efficiency to destroy bacterial and the pathogenic presence on the skin.

The hand disinfecting apparatus includes an electrical heater that receives the air flow from the ozone source, wherein the air flow includes ozone-enriched air and heats the received air flow including ozone-enriched air to generate an air flow including heated ozone-enriched air. The electrical heater as used herein refers to air heaters that heat the air and raise the temperature of the air to increase the efficiency of killing the bacteria and pathogens. Furthermore, the electrical heater may include hot air blowers, heaters, the like. In an example implementation, the electrical heater may increase temperature of the air (at a room temperature before heating) by 30 to 40 degrees Celsius, potentially more increase. Notably, the pathogens and the bacteria may not survive at the increased temperature and thus, be effectively eliminated.

The hand disinfecting apparatus includes the hand-receiving chamber that receives the one or more user's hands and passes the air flow including heated ozone-enriched air over the one or more user's hands. Furthermore, the hand-receiving chamber allows the user to insert their hand inside the hand disinfecting apparatus to pass the air flow including heated ozone-enriched air over the one or more user's hands. Moreover, the hand-receiving chamber may include a window or a door that allows insertion of the user's hands. Optionally, the hand-receiving chamber may include sensors to detect insertion and removal of user's hands inside the hand disinfecting apparatus.

The hand disinfecting apparatus includes a filter arrangement that receives the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow. The filter arrangement comprises dust- and particle-capturing materials such as porous or fibrous materials, that absorbs the bacteria and pathogens being released from the user's hands.

Optionally, the hand disinfecting apparatus further comprises an ultraviolet (UVC) source for disinfecting, or even sterilizing, an interior of the hand disinfecting apparatus. The ultraviolet (UVC) source as used herein refers to a deep ultraviolet generator that emits ultraviolet (UVC) rays for disinfection, or even sterilizing, the interior of the hand disinfecting apparatus; deep ultraviolet radiation (UVC) has a wavelength in a range of circa 180 nm to 250 nm, more optionally in a range of 205 nm to 230 nm. Deep ultra violet (UVC) sources include deep ultraviolet light emitting diodes (LEDs) and ultraviolet lasers; for example, a company Photon Systems Inc. (USA) has developed specialized deep UV sources ranging from ultra-narrow-linewidth deep UV laser emitting at 224.3 nm and 248.6 nm, as well as deep UV Light Emitting Diodes (LEDs) emitting in a 250 nm to 350 nm spectral range. Furthermore, the ultraviolet (UVC) rays are capable of destroying the pathogens and bacteria from the user's hands. Moreover, the ultraviolet (UVC) rays are introduced to the cleansing processing along with the hot air, to increase an efficiency of the hand disinfecting apparatus. Notably, the ultraviolet (UVC) source illuminates the ultraviolet rays at a preferred fluorophore light frequency.

Optionally, the hand disinfecting apparatus further comprises a control unit with data telemetry to a database for communicating an operating status or pathogen status of the hand disinfecting apparatus to a remote database. The control unit used herein refers to data processing units, smart controllers, and/or programmed devices that are operable to monitor and control the process of the hand disinfecting apparatus.

Optionally, the hand disinfecting apparatus further comprises a sensor arrangement for monitoring pathogen load on the filter arrangement, for recognizing pathogen type and communicating pathogen status via the control unit to the remote database. The sensor arrangement for monitoring pathogen load includes pathogen sensors that detect the pathogen type or the nature of pathogen. In an example implementation, the sensor arrangement may alarm on recognition of a dangerous pathogen type or a severe load of pathogens.

Optionally, the control unit further comprises a processing unit for managing operating sequences for the internal elements of the hand disinfecting apparatus. Furthermore, each of the steps that are associated with a hand disinfecting process of the user is managed through the processing unit. Moreover, the control unit is operable to control the order of the process and intensity of the processes involved in the cleansing of the user's hands.

Optionally, the control unit further comprises a computing arrangement for analysing pathogenic load in the filter arrangement using artificial intelligence (AI) or machine learning (ML) algorithms. It will be appreciated that the operating sequences for the internal elements of the hand disinfecting apparatus are automated and are controlled through programmed devices. Such devices may include computers, tablets, microprocessors, and the like.

Optionally, the ultraviolet source (UVC) further serves as an optical excitation source for interrogating polypeptide fluorophores that are applied as an aerosol mist to the filter arrangement, wherein the interrogating polypeptide fluorophores selectively bind to specific pathogens and are thereby used to monitor pathogenic load on the filter arrangement. Furthermore, the hand disinfecting apparatus diffuses the polypeptide fluorophores on the pathogens to detect the presence and to identify a type of pathogen (such as for example Ebola, Tuberculosis, and the like) thereof.

Optionally, the hand disinfecting apparatus further includes an imaging sensor for generating a temporal sequence of images of the filter arrangement to monitor its pathogenic load. It will be appreciated that the imaging sensor may collect the images of the user's hands before and after disinfection, for example sterilisation, to analyse the pathogenic load. Furthermore, the temporal sequence of images may provide a data analysis to control the internal elements accordingly. Moreover, the data analysis may be communicated over the server arrangement to monitor the pathogenic load and instruct the user to remove hands or continue for a certain time period based on the type of the pathogen detected.

Optionally, the hand disinfecting apparatus further includes a sprayer and/or an air blower. Furthermore, the sprayer may sprinkle a perfume, a hand sanitizer, to nourish the skin. Moreover, the air blower may soothe the skin after disinfection. In use, a given user will wash his/her hands in a disinfecting solution (for example, a sterilizing solution) and then insert his/her hands into the hand disinfecting apparatus for ensuring that all pathogens and microbes have been effectively killed.

The hand disinfecting apparatus includes a fan arrangement that provides for the air flow to be drawn from the top region to a bottom region of the hand disinfecting apparatus, wherein the fan arrangement includes an ozone absorbing arrangement. Furthermore, the fan arrangement as used herein refers to air blower for circulating the air from the top region to the bottom region. Moreover, the fan arrangement creates a pressure difference inside the hand disinfecting apparatus to draw the air from the top region to the bottom region. the ozone absorbing arrangement used herein refers to fibrous materials such as cotton, woollen, the like that traps the ozone and absorbs therein.

Pursuant to embodiment of the present disclosure, there is described a method of operating the hand disinfecting apparatus.

Optionally, the method further comprises acquiring information regarding a pathogen load by detecting pathogens on the filter arrangement by comparing differences in the temporal sequence of images, and communicating sensed pathogen load of the filter arrangement to the remote database. It will be appreciated that the temporal sequence of images is basis for recognizing the pathogen type and a load of the pathogens inside the hand disinfecting apparatus. More optionally, the method further comprises analysing pathogenic load in the filter arrangement using artificial intelligence and machine learning algorithms. The pathogenic load in the filter arrangement may be analysed from remote servers through control units.

Moreover, the present description also relates to the method as described above. The various embodiments and variants disclosed above apply *mutatis mutandis* to the method.

Optionally, the method further comprises disinfecting (for example, sterilizing) an interior of the hand disinfecting apparatus using the deep ultraviolet (UVC) source.

Optionally, the method further comprises communicating the operating status or pathogen status of the hand disinfecting apparatus using the control unit with data telemetry to the remote database.

Optionally, the method further comprises monitoring the pathogen load on the filter arrangement using the sensor arrangement, for recognizing pathogen type and communicating pathogen status via the control unit to the remote database.

Optionally, the method further comprises managing operating sequences for the internal elements of the hand disinfecting apparatus using the processing unit.

Optionally, the method further comprises analysing pathogenic load in the filter arrangement using Artificial Intelligence (AI) /Machine Learning (ML) algorithms via the computing arrangement.

Optionally, the method further comprises serving the deep ultraviolet (UVC) source as the optical excitation source for polypeptide fluorophores used to monitor pathogenic load on the filter arrangement.

### DETAILED DESCRIPTION OF THE DRAWINGS

Referring to FIGs. 1A, 1B, 1C, there is shown a schematic illustration of a hand disinfecting apparatus **100,** in accordance with an embodiment of the present disclosure. The hand disinfecting apparatus includes a housing **102** that includes an access arrangement **138** to receive one or more user's hands **130** into a hand-receiving chamber **140** of the hand disinfecting apparatus **100.** The hand disinfecting apparatus **100** includes an ozone source **110** to add ozone to an air flow through the ozone source **110;** optionally, the ozone source **110** is a proprietary commercial device using high electrical potentials to cause ionization of air to generate ozone. The hand disinfecting apparatus **100** also includes an electrical heater **112** that receives the air flow from the ozone source **110,** and heats the received air flow including ozone-enriched air to generate an air flow including heated ozone-enriched air. The hand disinfecting apparatus **100** includes the hand-receiving chamber **140** that receives the one or more user's hands **130** and passes the air flow including heated ozone-enriched air over the one or more user's hands **130.** The hand disinfecting apparatus **100** includes the filter arrangement **114** that receives the air flow of heated ozone-enriched air after it has passed over the one or more user's hands **130,** wherein the passed air flow including heated ozone-enriched air includes one or more pathogens released from the one or more user's hands **130,** wherein the filter arrangement **114** collects at least a portion of the one or more pathogens from the passed air flow. The hand disinfecting apparatus **100** also includes a fan arrangement **116** that provides for the air flow to be drawn (namely, sucked) in sequence from the ozone generator **110** via the electrical heater **112,** via the hand-receiving chamber **140** and via the filter arrangement **114** to the fan arrangement **116** of the hand disinfecting apparatus **100.**

The hand disinfecting apparatus **100,** when in operation, includes a top region **104** within the housing **102,** a central region **106** within the housing **102,** and a bottom region **108** within the housing **102.** In FIG. 1A, the ozone source **110** and the electrical heater are disposed in the top region **104,** and the filter arrangement **114** and the fan arrangement **116** are disposed in the bottom region **108;** such an arrangement provides for a slender elongate form to the hand disinfecting apparatus **100,** for example a floor-standing apparatus. In FIG. 1B, the ozone source **110** and the electrical heater **112** are disposed in the bottom region **108,** and the filter arrangement **114** and the fan arrangement **116** are disposed in the top region **104;** such an arrangement also provides for a slender elongate form to the hand disinfecting apparatus **100,** for example a floor-standing apparatus. In FIG. 1C, the ozone source **110** and the electrical heater are disposed in first lateral side region **132** relative to the central region **106,** and the filter arrangement **114** and the fan arrangement **116** are disposed in a second lateral region **134** relative to the central region **106;** such an arrangement also provides for a compact lateral form to the hand disinfecting apparatus **100,** for example implemented as a table-top standing apparatus.

The hand disinfecting apparatus **100** optionally comprising a deep ultraviolet (UVC) source **120** for disinfecting (for example, sterilizing) an interior of the hand disinfecting apparatus **100.**

Optionally, the ultraviolet source (UVC) **120** further serves as an optical excitation source for interrogating polypeptide fluorophores that are applied as an aerosol mist to the filter arrangement **114,** wherein the interrogating polypeptide fluorophores selectively bind to specific pathogens and are thereby used to monitor pathogenic load on the filter arrangement **114.** Furthermore, the hand disinfecting apparatus **100** diffuses the polypeptide fluorophores on the pathogens to detect the presence and to identify a type of pathogen (such as for example Ebola, Tuberculosis, and the like) thereof.

Optionally, the hand disinfecting apparatus **100** includes a sensor, for example coupled to a control unit **118** that manages operation of the hand disinfecting apparatus **100,** that switches off the electrical heater **112** after a hand disinfecting process has been completed, and initiates a circulation of ozone-enriched air into a room (not shown) accommodating the hand disinfecting apparatus **100,** when there are no people present in the room, so that the room will be cleaned of pathogens occasionally between the use of the room by the people.

Referring to FIG. 2, there is shown a process-time graph **200** of a sequence of activating component parts of the hand disinfecting apparatus of FIG. 1 when in use to dry and disinfect a user's hands using the hand disinfecting apparatus **100,** in accordance with an embodiment of the present disclosure. As shown, the graph **202** depicts the insertion and removal of user's hands **106.** The graph **204** also depicts addition of ozone **110.** The graph **206** also depicts execution of fan arrangement **116.** The graph **208** also depicts excitation of the ultraviolet (UVC) source **120.** When disinfecting the room accommodating the hand disinfecting apparatus **100,** it will be appreciated that the ozone source **110,** the fan arrangement **116** and optionally the deep ultraviolet (UVC) source **120** are maintained in operation over an extensive period (for example minutes or hours) after use of the hand disinfecting apparatus **100** by a given user; in such a situation, the fan arrangement **116** directs an ozone-enriched air flow into the room (for example, via an orifice or port in the housing **102** provided with actuated baffle).

Referring to FIG. 3, there are shown steps of a method **300** of operating a hand disinfecting apparatus, in accordance with an embodiment of the present disclosure. At a step **302,** an ozone source disposed at a top region of the hand disinfecting apparatus is used to add ozone to an air flow through the ozone source. At a step **304,** an electrical heater is used that receives the air flow from the ozone source, wherein the air flow includes ozone-enriched air and heats the received air flow including ozone-enriched air, to generate an air flow including heated ozone-enriched air. At a step **306,** the hand-receiving chamber is used to receive the one or more user's hands and to pass the air flow including heated ozone-enriched air over the one or more user's hands. At a step **308,** a filter arrangement is used to receive the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow. At a step **310,** a fan arrangement is used to provide for the air flow from the top region to a bottom region of the hand disinfecting apparatus, wherein the fan arrangement includes an ozone absorbing arrangement.

It will be appreciated that, when the hand disinfecting apparatus **100** is to be used, a given user washes his or her hands in clean water for circa 30 seconds, and then inserts his or hands into the hand-receiving chamber **140** of the hand disinfecting apparatus **100** for a period of circa 30 seconds, during which a heated ozone-enriched air flow dries and disinfects the hands.

The steps **302** to **310** are only illustrative and other alternatives can also be provided where one or more steps are added, one or more steps are removed, or one or more steps are provided in a different sequence without departing from the scope of the claims herein.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A hand disinfecting apparatus including a housing, wherein the housing includes a central region having an access arrangement to receive one or more user's hands into a hand-receiving chamber of the hand disinfecting apparatus, **characterized in that**
when the hand disinfecting apparatus, when deployed for operation, has its internal elements disposed, from a first region within the housing to a second region within the housing:
(a) an ozone source at the first region within the housing of the hand disinfecting apparatus that adds ozone to an air flow through the ozone source;
(b) an electrical heater that receives the air flow from the ozone source, wherein the air flow includes ozone-enriched air and heats the received air flow including ozone-enriched air to generate an air flow including heated ozone-enriched air;
(c) the hand-receiving chamber that receives the one or more user's hands and passes the air flow including heated ozone-enriched air over the one or more user's hands;
(d) a filter arrangement that receives the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) a fan arrangement that provides for the air flow that is drawn from the first region to the region second of the hand disinfecting apparatus, wherein the fan arrangement includes an ozone absorbing arrangement.

2. A hand disinfecting apparatus of claim 1, **characterized in that** the first region is a top region of the housing and the second region is a bottom region of the housing, when the hand disinfecting apparatus is in operation.

3. A hand disinfecting apparatus (100) of any one of the preceding claims, **characterized in that** further comprising a deep ultraviolet (UVC, 120) source for disinfecting an interior of the hand disinfecting apparatus (100) .

4. A hand disinfecting apparatus (100) of any one of the preceding claims, **characterized in that** the hand disinfecting apparatus (100) includes a sensor (e.g. ultrasonic motion sensor or infra-red motion sensor) that switches off power applied to the electrical heater (112) after a hand disinfecting process has been completed, and initiates a circulation of ozone-enriched air into a room accommodating the hand disinfecting apparatus (100), when there are people are absent from the room, to clean the room of pathogens between a use of the room by the people.

5. A hand disinfecting apparatus of any one of the preceding claims, **characterized in that** the hand disinfecting apparatus further comprises a control unit with data telemetry to a database for communicating an operating status or pathogen status of the hand disinfecting apparatus to a remote database.

6. A hand disinfecting apparatus of claim 5, **characterized in that** further comprising a sensor arrangement for monitoring pathogen load on the filter arrangement, for recognizing pathogen type and communicating pathogen status via the control unit to a remote database.

7. A hand disinfecting apparatus of claim 5, **characterized in that** the control unit further comprises a processing unit for managing operating sequences for internal elements of the hand disinfecting apparatus.

8. A hand disinfecting apparatus of claim 5, **characterized in that** the control unit further comprises a computing arrangement for analysing pathogenic load in the filter arrangement using Artificial Intelligence (AI)/Machine Learning (ML) algorithms.

9. A hand disinfecting apparatus of claim 3, **characterized in that** the deep ultraviolet (UVC) source further serves as an optical excitation source for polypeptide fluorophores used to monitor pathogenic load on the filter arrangement.

10. A hand disinfecting apparatus of any one of the preceding claims, **characterized in that** further includes an imaging sensor for generating a temporal sequence of images of the filter arrangement to monitor its pathogenic load.

11. A method of operating a hand disinfecting apparatus including a housing, wherein the housing includes in a central region within the housing an access arrangement to receive one or more user's hands into a hand-receiving chamber of the hand disinfecting apparatus, **characterized in that**
the method includes steps of:
(a) using an ozone source disposed at a first region within the housing of the hand disinfecting apparatus to add ozone to an air flow through the ozone source;
(b) using an electrical heater that receives the air flow from the ozone source, wherein the air flow includes ozone-enriched air and heats the received air flow including ozone-enriched air, to generate an air flow including heated ozone-enriched air;
(c) using the hand-receiving chamber to receives the one or more user's hands and to pass the air flow including heated ozone-enriched air over the one or more user's hands;
(d) using a filter arrangement to receive the air flow of heated ozone-enriched air after it has passed over the one or more user's hands, wherein the passed air flow including heated ozone-enriched air includes one or more pathogens released from the one or more user's hands, wherein the filter arrangement collects at least a portion of the one or more pathogens from the passed air flow; and
(e) using a fan arrangement to provide for the air flow to be drawn from the first region to a second region within the housing of the hand disinfecting apparatus, wherein the fan arrangement includes an ozone absorbing arrangement.

12. A method according to claim 11, the method further comprising acquiring a measure of pathogen load by:
- detecting pathogens on the filter arrangement by comparing differences in the temporal sequence of images, and communicating the sensed pathogen load of the filter arrangement to the remote database; and
- analysing the pathogenic load in the filter arrangement using Artificial Intelligence/Machine Learning algorithms.

13. A method according to claim 11 or 12, **characterized in that** the method further comprising disinfecting an interior of the hand disinfecting apparatus using an ultraviolet (UVC) source.

14. A method according to claim 12 or 13, **characterized in that** the method further comprising communicating an operating status or pathogen status of the hand disinfecting apparatus using a control unit with data telemetry to a remote database.

15. A method according to claim 13, **characterized in that** the method further comprising using the deep ultraviolet (UVC) source as the optical excitation source for polypeptide fluorophores used to monitor pathogenic load on the filter arrangement.
